# EUROPEAN PATENT SPECIFICATION

(11) **EP 3 251 581 B1**
(45) Date of publication and mention of the grant of the patent: **19.02.2020**
(21) Application number: 16743160.0
(22) Date of filing: 18.01.2016
(51) Int. Cl.: A61B 1/00, A61B 1/04, A61B 1/05, A61B 5/00

(54) **PROCESSOR DEVICE FOR ENDOSCOPE, METHOD FOR OPERATING SAME, AND CONTROL PROGRAM**
PROZESSORVORRICHTUNG FÜR EIN ENDOSKOP, VERFAHREN ZUM BETRIEB DAVON UND STEUERUNGSPROGRAMM
DISPOSITIF DE PROCESSEUR POUR ENDOSCOPE, SON PROCÉDÉ DE FONCTIONNEMENT, ET PROGRAMME DE COMMANDE

(30) Priority: 26.01.2015 JP 2015012582
(43) Date of publication of application: 06.12.2017
(73) Proprietor: FUJIFILM Corporation, Tokyo 106-8620 (JP)
(72) Inventor: KAKU, Toshihiko, Ashigarakami-gun Kanagawa 258-8538 (JP)
(74) Representative: Jeffrey, Philip Michael
(86) International application number: PCT/JP2016/051299
(87) International publication number: WO 2016/121556

(56) References cited:
- EP-A1- 2 505 140
- WO-A1-2013/121610
- JP-A- H09 294 708
- JP-A- 2003 199 707
- JP-A- 2012 213 552
- US-A1- 2009 270 702

## Description

### Field of the Invention

The present invention relates to a processor device for an endoscope for calculating blood vessel index values, such as a blood vessel density, a blood vessel thickness, and a blood vessel length, an operation method thereof, and a non-transitory computer readable medium.

### Description of the Related Art

In the medical field, diagnosis using an endoscope system including a light source device, an endoscope, and a processor device has been widely performed. The light source device emits illumination light for illuminating an observation target. The endoscope images the observation target illuminated with the illumination light using an imaging sensor, and outputs an image signal. The processor device generates an image of the observation target from the image signal transmitted from the endoscope, and displays the image on a monitor.

In the diagnosis using the endoscope system, there is a demand to acquire a distance between the observation target and the distal end portion of the endoscope (hereinafter, referred to as an observation distance) for various reasons. For example, in JP2012-213552A, in order to generate an image suitable for the purpose of diagnosis, the observation distance is acquired by analyzing the frequency of the image signal. There are other methods for acquiring the observation distance. For example, it is possible to acquire the observation distance from the distance scale of a scale inserted into the forceps port, acquire the observation distance from the amount of exposure obtained from the image signal, or acquire the observation distance from a change in imaging magnification in the case of moving the zoom lens.

### SUMMARY OF THE INVENTION

### Problems to Be Solved by the Invention

However, it is often difficult to acquire the observation distance depending on the imaging conditions. For example, assuming that there is movement in the observation target, blood vessels in the image are likely to be blurred. Accordingly, in the frequency analysis disclosed in JP2012-213552A, there is a possibility that high frequency components will collapse. For this reason, it is often difficult to acquire the observation distance accurately. In addition, in the case of acquiring the observation distance from the scale, time and effort to insert the scale into the forceps port are required. In the case of acquiring the observation distance from the amount of exposure, assuming that there is a foreign matter or the like on the surface of the observation object, the reflectance of illumination light changes. Accordingly, it may be difficult to acquire the observation distance accurately. In the case of acquiring the observation distance from the imaging magnification, it is necessary to change the imaging magnification. Accordingly, it is not possible to acquire the observation distance at the time of non-magnification.

It is an object of the present invention to provide a processor device for an endoscope capable of accurately acquiring an observation distance, an operation method thereof, and a non-transitory computer readable medium.

### Means for Solving the Problems

In order to achieve the aforementioned object, a processor device for an endoscope of the present invention comprises: an image signal acquisition unit, a blood vessel index value calculation unit, and an observation distance calculation unit. The image signal acquisition unit acquires an image signal obtained by imaging an observation target with the endoscope. The blood vessel index value calculation unit calculates at least one or more blood vessel index values, among the number of blood vessels, a length of a blood vessel, a difference in blood vessel height, an inclination of a blood vessel, an area of a blood vessel, a density of blood vessels, a depth of a blood vessel, and a gap between blood vessels that are blood vessel index values of blood vessels contained in the observation target, from the image signal. The observation distance calculation unit calculates an observation distance between a distal end of the endoscope and a surface of the observation target from at least one or more blood vessel index values of the blood vessel index values calculated by the blood vessel index value calculation unit.

An operation method of a processor device for an endoscope of the present invention comprises : a step in which an image signal acquisition unit acquires an image signal obtained by imaging an observation target with the endoscope; a step in which a blood vessel index value calculation unit calculates at least one or more blood vessel index values, among the number of blood vessels, a length of a blood vessel, a difference in blood vessel height, an inclination of a blood vessel, an area of a blood vessel, a density of blood vessels, a depth of a blood vessel, and a gap between blood vessels that are blood vessel index values of blood vessels contained in the observation target, from the image signal; and a step in which an observation distance calculation unit calculates an observation distance between a distal end of the endoscope and a surface of the observation target from at least one or more blood vessel index values of the blood vessel index values calculated by the blood vessel index value calculation unit.

A non-transitory computer readable medium for storing a control program for an endoscope that is installed in a processor device for the endoscope of the present invention causes a computer to function as an image signal acquisition unit, a blood vessel index value calculation unit, and an observation distance calculation unit. The image signal acquisition unit acquires an image signal obtained by imaging an observation target with the endoscope. The blood vessel index value calculation unit calculates at least one or more blood vessel index values, among the number of blood vessels, a length of a blood vessel, a difference in blood vessel height, an inclination of a blood vessel, an area of a blood vessel, a density of blood vessels, a depth of a blood vessel, and a gap between blood vessels that are blood vessel index values of blood vessels contained in the observation target, from the image signal. The observation distance calculation unit calculates an observation distance between a distal end of the endoscope and a surface of the observation target from at least one or more blood vessel index values of the blood vessel index values calculated by the blood vessel index value calculation unit.

It is preferable that the blood vessel index value calculation unit further calculates a thickness of a blood vessel as the blood vessel index value and that the observation distance calculation unit calculates the observation distance from at least two or more of the blood vessel index values among the blood vessel thickness and blood vessel index values other than the blood vessel thickness.

It is preferable to further comprise a blood vessel index value selection unit that selects the blood vessel index value used in calculation of the observation distance by the observation distance calculation unit among the blood vessel index values.

It is preferable to further comprise a priority setting unit that sets a priority for each of the blood vessel index values . It is preferable that the blood vessel index value selection unit selects the blood vessel index value, for which the priority set by the priority setting unit satisfies specific conditions, as the blood vessel index value used in calculation of the observation distance.

It is preferable that the observation distance calculation unit sets an average value, which is obtained by averaging the observation distance for each of the blood vessel index values, as the observation distance.

It is preferable to further comprise a reference storage unit that stores a predetermined reference observation distance and a reference blood vessel index value at the reference observation distance. It is preferable that the observation distance calculation unit calculates the observation distance from the blood vessel index value calculated by the blood vessel index value calculation unit, the reference blood vessel index value, and the reference observation distance.

It is preferable that the blood vessel index values include a distance-invariant blood vessel index value and that the observation distance calculation unit calculates the observation distance from the blood vessel index value other than the distance-invariant blood vessel index value. It is preferable that the distance-invariant blood vessel index value is invariant with respect to the observation distance. It is preferable that the distance-invariant blood vessel index value is the density.

### Effect of the Invention

According to the present invention, since the observation distance is calculated from the blood vessel index value calculated from the image signal, it is possible to provide a processor device for an endoscope capable of accurately acquiring the observation distance, an operation method thereof, and a control program.

### BRIEF DESCRIPTION OF THE DRAWINGS

Fig. 1 is an external view of an endoscope system.
Fig. 2 is a block diagram showing a function of the endoscope system.
Fig. 3 is a graph showing the spectra of violet light, blue light, green light, and red light.
Fig. 4 is a graph showing the spectral characteristics of a color filter.
Fig. 5 is a block diagram showing the configuration of a measurement image processing unit.
Fig. 6 is a schematic diagram showing a blood vessel index value in an ROI.
Fig. 7 is a schematic diagram of an observation distance calculation table.
Fig. 8 is a schematic diagram of a monitor on which information indicating a blood vessel index value and an observation distance are displayed.
Fig. 9 is a flowchart of a first embodiment.
Fig. 10 is a block diagram showing another configuration of the measurement image processing unit.
Fig. 11 is a block diagram showing the configuration of a reference storage section.
Fig. 12 is a schematic diagram of a monitor on which information indicating that there is a possibility of a lesion is displayed.
Fig. 13 is a block diagram showing a function of an endoscope system of a second embodiment.
Fig. 14 is a graph showing the emission spectrum of white light.
Fig. 15 is a graph showing the emission spectrum of special light.
Fig. 16 is a block diagram showing a function of an endoscope system of a third embodiment.
Fig. 17 is a plan view showing a rotary filter.

### DESCRIPTION OF THE PREFERRED EMBODIMENTS

### [First embodiment]

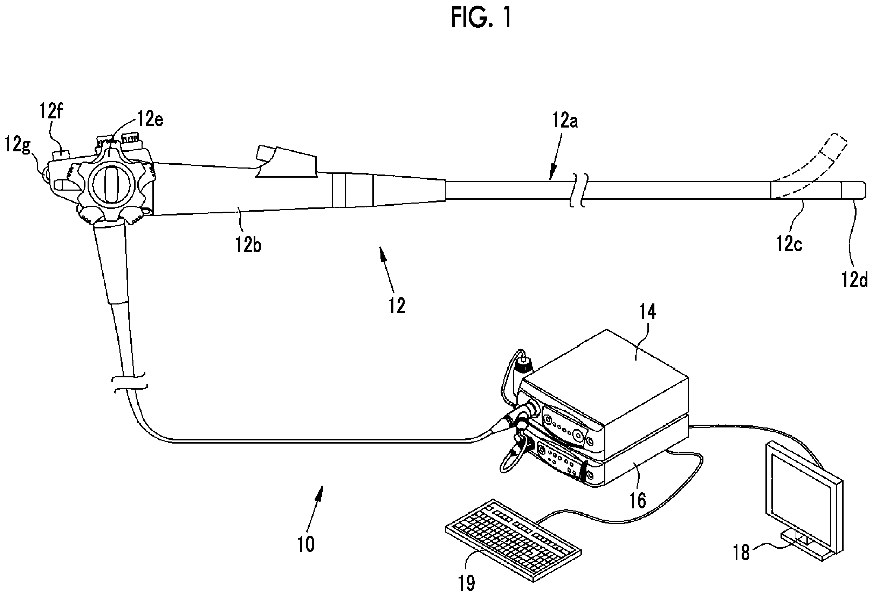

As shown in Fig. 1, an endoscope system 10 includes an endoscope 12, a light source device 14, a processor device 16, a monitor 18, and a console 19. The endoscope 12 is optically connected to the light source device 14, and is electrically connected to the processor device 16. The endoscope 12 includes an insertion part 12a that is inserted into the body of an observation target, an operation unit 12b provided in the proximal end portion of the insertion part 12a, and a bending portion 12c and a distal end portion 12d that are provided at the distal end side of the insertion part 12a. By operating an angle knob 12e of the operation unit 12b, the bending portion 12c is bent. Through the bending operation, the distal end portion 12d is directed in a desired direction.

In addition to the angle knob 12e, a mode selector switch (mode selector SW) 12f and a zoom operation unit 12g are provided in the operation unit 12b. The mode selector SW 12f is used for an observation mode switching operation. The endoscope system 10 has a normal mode and a measurement mode as observation modes. In the normal mode, an image of natural colors obtained by imaging an observation target using white light as illumination light (hereinafter, referred to as a normal image) is displayed on the monitor 18. In the measurement mode, a blood vessel index value of a blood vessel contained in the observation target is calculated, an observation distance is acquired from the blood vessel index value, and an image based on the blood vessel index value (hereinafter, referred to as a special image) is displayed on the monitor 18. The zoom operation unit 12g is used to input an imaging magnification change instruction for instructing an imaging optical system 30b, which will be described later, to change the imaging magnification.

The processor device 16 is electrically connected to the monitor 18 and the console 19. The monitor 18 outputs and displays an image of the observation target, information attached to the image of the observation target, and the like. The console 19 functions as a user interface for receiving an input operation, such as function setting. In addition, an external recording unit (not shown) in which an image, image information, and the like are recorded may be connected to the processor device 16.

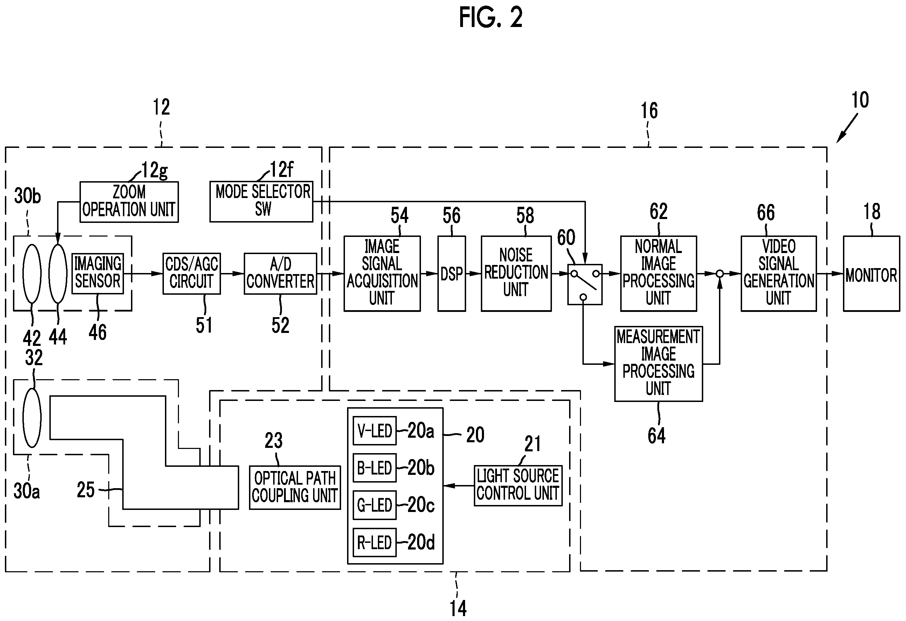

As shown in Fig. 2, the light source device 14 includes a light source 20 and a light source control unit 21 that controls the light source 20. The light source 20 emits illumination light for illuminating the observation target. For example, the light source 20 has a plurality of semiconductor light sources, and turns on or off each of the semiconductor light sources and generates illumination light by controlling the amount of light emitted from each semiconductor light source in the case of turning on each semiconductor light source. In the present embodiment, the light source unit 20 includes four LEDs of a violet light emitting diode (V-LED) 20a, a blue light emitting diode (B-LED) 20b, a green light emitting diode (G-LED) 20c, and a red light emitting diode (R-LED) 20d.

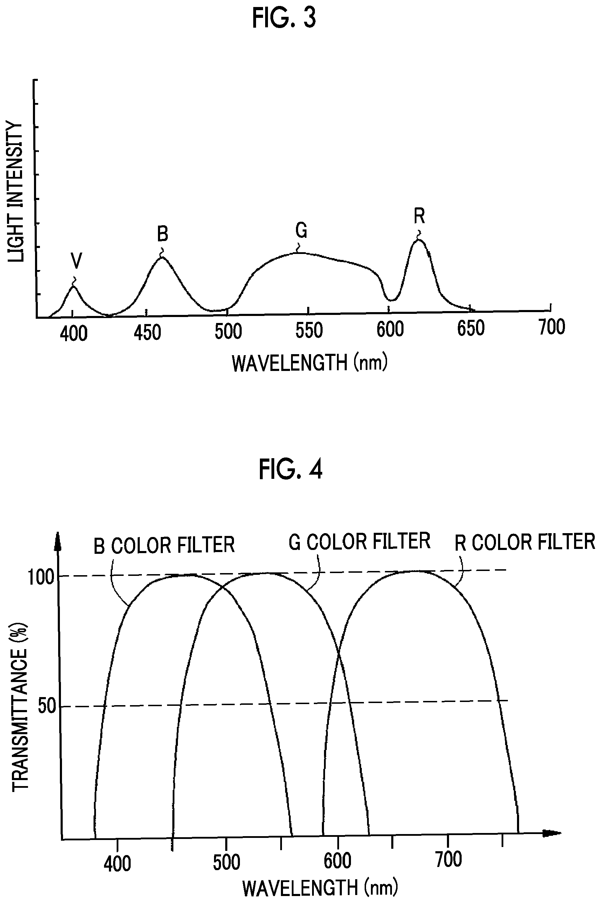

As shown in Fig. 3, the V-LED 20a is a violet semiconductor light source that emits violet light V in a wavelength band of 380 nm to 420 nm that has a center wavelength of 405 nm. The B-LED 20b is a blue semiconductor light source that emits blue light B in a wavelength band of 420 nm to 500 nm that has a center wavelength of 460 nm. The G-LED 20c is a green semiconductor light source that emits green light G having a wide wavelength band of 480 nm to 600 nm. The R-LED 20d is a red semiconductor light source that emits red light R in a wavelength band of 600 nm to 650 nm that has a center wavelength of 620 nm to 630 nm. In addition, the center wavelengths of the violet light V and the blue light B have a width of about +5 nm to +10 nm.

The light source control unit 21 independently controls ON or OFF of each of the LEDs 20a to 20d, the amount of light emission at the time of lighting, and the like by inputting a control signal to each of the LEDs 20a to 20d. In the present embodiment, in both observation modes of the normal mode and the measurement mode, the light source control unit 21 turns on all of the V-LED 20a, the B-LED 20b, the G-LED 20c, and the R-LED 20d. Therefore, white light including the violet light V, the blue light B, the green light G, and the red light R is used as illumination light in the normal mode and the measurement mode.

Light of each color emitted from each of the LEDs 20a to 20d is incident on a light guide 25, which is inserted into the insertion part 12a, through an optical path coupling unit 23 formed of a mirror, a lens, or the like. The light guide 25 is built into the endoscope 12 and the universal cord. The universal cord connects the endoscope 12 with the light source device 14 and the processor device 16. The illumination light emitted from the light source 20 propagates to the distal end portion 12d of the endoscope 12 through the light guide 25.

An illumination optical system 30a and the imaging optical system 30b are provided in the distal end portion 12d of the endoscope 12. The illumination optical system 30a has an illumination lens 32. The illumination light propagated from the light source 20 by the light guide 25 is emitted to the observation target through the illumination lens 32. The imaging optical system 30b includes an objective lens 42, a zoom lens 44, and an imaging sensor 46. Various kinds of light, such as reflected light, scattered light, and fluorescence from the observation target, are incident on the imaging sensor 46 through the objective lens 42 and the zoom lens 44. As a result, an image of the observation target is formed on the imaging sensor 46.

The zoom lens 44 freely moves between the telephoto end and the wide end based on the imaging magnification change instruction from the zoom operation unit 12g, thereby making it possible to change the imaging magnification. In the event that the zoom lens 44 moves to the wide end, the image of the observation target is magnified. On the other hand, in the event that the zoom lens 44 moves to the telephoto end, the image of the observation target is reduced. In order that non-magnified observation is performed, the zoom lens 44 is disposed at the wide end. In order that magnified observation is performed by operating the zoom operation unit 12g, the zoom lens 44 moves from the wide end to the telephoto end.

The imaging sensor 46 is a color imaging sensor, and images the observation target illuminated with the illumination light and outputs an image signal. One of a blue (B) color filter, a green (G) color filter, and a red (R) color filter shown in Fig. 4 is provided in each pixel of the imaging sensor 46. Accordingly, the imaging sensor 46 receives violet light to blue light in a B pixel (blue pixel) in which a B color filter is provided, receives green light in a G pixel (green pixel) in which a G color filter is provided, and receives red light in an R pixel (red pixel) in which an R color filter is provided. Then, the image signal of each color of RGB is output from the pixel of each color.

As the imaging sensor 46, it is possible to use a charge coupled device (CCD) imaging sensor or a complementary metal oxide semiconductor (CMOS) imaging sensor. Instead of the primary color imaging sensor 46, a complementary color imaging sensor including complementary color filters of cyan (C), magenta (M), yellow (Y), and green (G) may be used. In the case of using the complementary color imaging sensor, image signals of four colors of CMYG are output. Therefore, by converting the image signals of four colors of CMYG into image signals of three colors of RGB by complementary color-primary color conversion, it is possible to obtain the same image signals of RGB colors as in the imaging sensor 46. Instead of the imaging sensor 46, a monochrome imaging sensor in which no color filter is provided may be used.

A correlated double sampling/automatic gain control (CDS/AGC) circuit 51 performs correlated double sampling (CDS) or automatic gain control (AGC) for the analog image signal obtained from the imaging sensor 46. The analog image signal having passed through the CDS/AGC circuit 51 is converted into a digital image signal by an analog/digital (A/D) converter 52. The digital image signal after A/D conversion is input to the processor device 16.

The processor device 16 includes an image signal acquisition unit 54, a digital signal processor (DSP) 56, a noise reduction unit 58, an image processing switching unit 60, a normal image processing unit 62, a measurement image processing unit 64, and a video signal generation unit 66. The image signal acquisition unit 54 acquires a digital image signal from the imaging sensor 46 through the CDS/AGC circuit 51 and the A/D converter 52.

The DSP 56 performs various kinds of signal processing, such as defect correction processing, offset processing, gain correction processing, linear matrix processing, gamma conversion processing, and demosaic processing, on the acquired image signals. The defect correction processing is for correcting the signal of a defective pixel of the imaging sensor 46. The offset processing is for setting an accurate zero level by removing a dark current component from the image signal subjected to the defect correction processing. The gain correction processing is for adjusting the signal level by multiplying the image signal subjected to the offset processing by a specific gain.

The linear matrix processing increases the color reproducibility of the image signal subjected to the gain correction processing. Then, the gamma conversion processing is for adjusting the brightness or saturation of the image signal subjected to the linear matrix processing. The demosaic processing (also referred to as isotropic processing or synchronization processing) is for generating a signal of color lacking in each pixel of the image signal subjected to the gamma conversion processing by interpolation. Through the demosaic processing, all pixels have signals of RGB colors. The noise reduction unit 58 reduces noise by performing noise reduction processing (using, for example, a moving average method or a median filter method) on the image signal subjected to the demosaic processing or the like by the DSP 56. The image signal subjected to the noise reduction processing is transmitted to the image processing switching unit 60.

The image processing switching unit 60 transmits the image signal to the normal image processing unit 62 in a case where the normal mode is set by operating the mode selector SW 12f, and transmits the image signal to the measurement image processing unit 64 in a case where the measurement mode is set.

The normal image processing unit 62 generates a normal image by performing color conversion processing, color enhancement processing, and structure enhancement processing on the image signal received from the image processing switching unit 60. In the color conversion processing, color conversion processing, such as 3x3 matrix processing, gradation transformation processing, and three-dimensional look-up table (LUT) processing, is performed on the image signal. The color enhancement processing is performed on the image signal subjected to the color conversion processing. The structure enhancement processing is, for example, processing for enhancing the structure of the observation target, such as a blood vessel or a pit pattern (gland duct), and is performed on the image signal after the color enhancement processing. A color image using the image signal subjected to various kinds of image processing and the like as described above is a normal image.

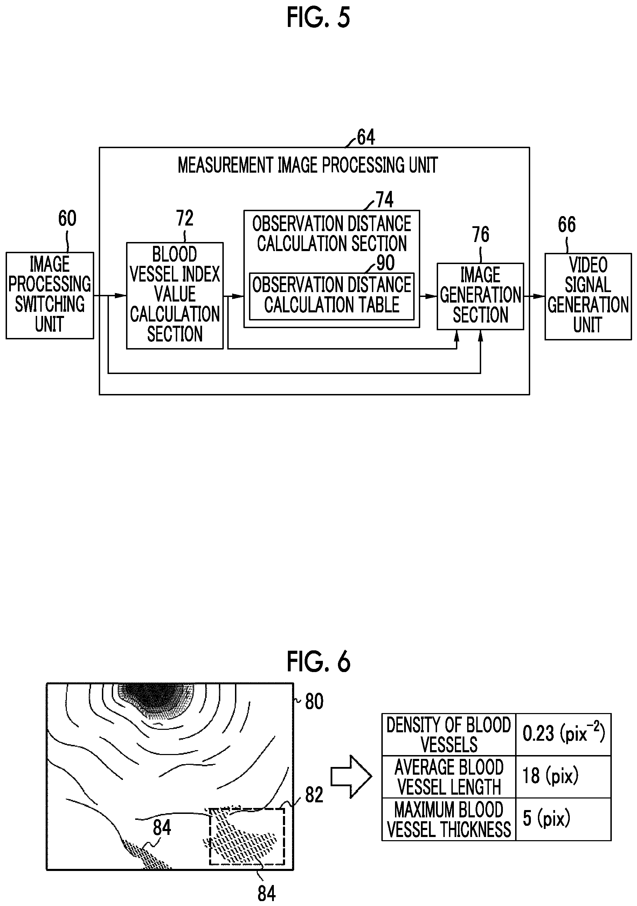

The measurement image processing unit 64 calculates a blood vessel index value by indexing (digitizing) the blood vessel indicated by the image signal received from the image processing switching unit 60, and calculates an observation distance that is the distance between the distal end portion 12d of the endoscope 12 and the surface of the observation target. As shown in Fig. 5, a blood vessel index value calculation section 72, an observation distance calculation section 74, and an image generation section 76 are provided in the measurement image processing unit 64. The image signal from the image processing switching unit 60 is received by the blood vessel index value calculation section 72 and the image generation section 76.

The blood vessel index value calculation section 72 calculates a blood vessel index value from the image signal received from the image processing switching unit 60. Blood vessel index values are the number of blood vessels contained in the observation target, the length of a blood vessel (or the average blood vessel length that is the average value of the lengths of blood vessels contained in the observation target), the thickness of a blood vessel (or a maximum blood vessel thickness that is the maximum value of the thicknesses of blood vessels contained in the observation target), a difference in blood vessel height, the inclination of a blood vessel, the area of a blood vessel, the density of blood vessels (proportion of blood vessels in unit area), the depth of a blood vessel, a gap between blood vessels, and the like. The blood vessel index value calculation section 72 calculates at least one or more of these blood vessel index values from the image signal. The type of the blood vessel index value calculated by the blood vessel index value calculation section 72 can be selected by inputting it using a user interface, such as the console 19, for example. In the present embodiment, a blood vessel density, an average blood vessel length, and a maximum blood vessel thickness are calculated as blood vessel index values. The average blood vessel length and the maximum blood vessel thickness are indicated by the number of pixels (pixel: pix) indicating blood vessels on the screen, and the density of blood vessels is indicated by (pix⁻²). The blood vessel index value calculation section 72 transmits the calculated blood vessel index value to the observation distance calculation section 74.

Specifically, in Fig. 6, in the case of calculating the density of blood vessels, the blood vessel index value calculation section 72 cuts out a region having a specific size (unit area) within a region of interest (ROI) 82 set for an image signal 80, and calculates a proportion of blood vessels 84 occupied in all the pixels within the region. By performing this on all the pixels within the ROI 82, the blood vessel index value calculation section 72 calculates the density of blood vessels 84 in each pixel of the ROI 82. In the case of calculating the average blood vessel length, the blood vessel index value calculation section 72 calculates the length of the blood vessel 84 in each pixel of the ROI 82, and calculates the average value of the length of the blood vessel 84 in each pixel. In the case of calculating the maximum blood vessel thickness, the blood vessel index value calculation section 72 calculates the thickness of the blood vessel 84 in each pixel of the ROI 82, and calculates the maximum value of the thickness of the blood vessel 84 in each pixel. In the present embodiment, the density of blood vessels is set to DB (pix⁻²), the average blood vessel length is set to AL (pix), and the maximum blood vessel thickness is set to MD (pix). For example, the density of blood vessels is set to DB = 0.23 (pix⁻²), the average blood vessel length is set to AL = 18 (pix), and the maximum blood vessel thickness is set to MD = 5 (pix). In addition to these blood vessel index values, the blood vessel index value calculation section 72 counts the number of blood vessels in each pixel in the image signal, for example, in the case of calculating the number of blood vessels.

The observation distance calculation section 74 calculates the observation distance from the blood vessel index values received from the blood vessel index value calculation section 72. Specifically, the observation distance calculation section 74 calculates the observation distance from blood vessel index values other than a distance-invariant blood vessel index value among the blood vessel index values calculated by the blood vessel index value calculation section 72. The distance-invariant blood vessel index value is not changed even in a case where the size (length, thickness, or the like) of the blood vessel in an image is changed according to the observation distance. That is, the distance-invariant blood vessel index value is a blood vessel index value that is invariant with respect to the observation distance. The observation distance calculation section 74 determines whether or not the blood vessel index value received from the blood vessel index value calculation section 72 is a blood vessel index value other than the distance-invariant blood vessel index value. The observation distance calculation section 74 calculates the observation distance from the average blood vessel length and the maximum blood vessel thickness among the density of blood vessels, the average blood vessel length, and the maximum blood vessel thickness calculated by the blood vessel index value calculation section 72.

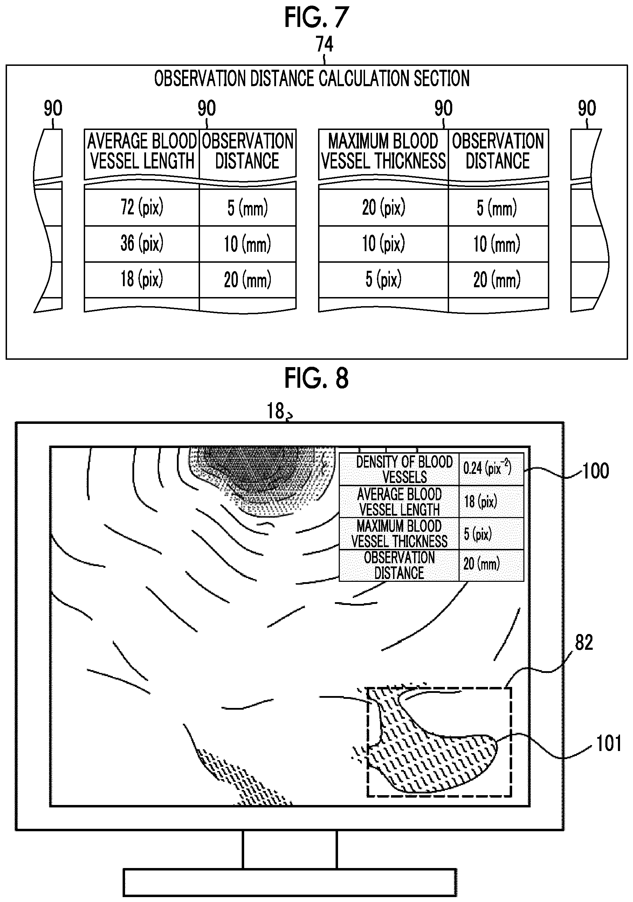

As shown in Fig. 7, the observation distance calculation section 74 has an observation distance calculation table 90 in which a blood vessel index value and an observation distance obtained from the blood vessel index value are associated with each other. Then, the observation distance calculation section 74 acquires the observation distance corresponding to the blood vessel index value received from the blood vessel index value calculation section 72 among the blood vessel index values in the observation distance calculation table 90. For example, since the average blood vessel length AL received from the blood vessel index value calculation section 72 is 18 (pix), the observation distance calculation section 74 acquires 20 (mm) as the observation distance corresponding to the average blood vessel length of 18 (pix) in the observation distance calculation table 90 in which the average blood vessel length and the observation distance are associated with each other. In addition, since the maximum blood vessel thickness MD received from the blood vessel index value calculation section 72 is 5 (pix), the observation distance calculation section 74 acquires 20 (mm) as the observation distance corresponding to the maximum blood vessel thickness of 5 (pix) in the observation distance calculation table 90 in which the maximum blood vessel thickness and the observation distance are associated with each other. In a case where a plurality of observation distances are acquired from a plurality of blood vessel index values, the observation distance calculation section 74 acquires an average value by averaging the observation distances. In the present embodiment, since both the observation distance corresponding to the average blood vessel length and the observation distance corresponding to the maximum blood vessel thickness are 20 (mm), the average value thereof is 20 (mm).

In the observation distance calculation table 90, in a case where there is no blood vessel index value calculated by the blood vessel index value calculation section 72, the observation distance calculation section 74 calculates the observation distance using the following methods. First, the observation distance calculation section 74 selects a blood vessel index value closest to the blood vessel index value calculated by the blood vessel index value calculation section 72, among the blood vessel index values in the observation distance calculation table 90, and calculates a ratio between the selected blood vessel index value and the blood vessel index value calculated by the blood vessel index value calculation section 72. Then, the observation distance calculation section 74 acquires an observation distance corresponding to the selected blood vessel index value with reference to the observation distance calculation table 90, multiplies the acquired observation distance by the above-described ratio, and sets the multiplication result as the observation distance corresponding to the blood vessel index value calculated by the blood vessel index value calculation section 72.

For example, in a case where the maximum blood vessel thickness MD calculated by the blood vessel index value calculation section 72 is 16 (pix), the observation distance calculation section 74 selects 20 (pix) as a maximum blood vessel thickness closest to the maximum blood vessel thickness MD = 16 (pix) in the observation distance calculation table 90 in which the maximum blood vessel thickness and the observation distance are associated with each other. The observation distance calculation section 74 calculates the ratio between the selected maximum blood vessel thickness and the selected maximum blood vessel thickness MD as 20 (pix)/16 (pix) = 1.25. Then, the observation distance calculation section 74 acquires 5 (mm) as the observation distance corresponding to the selected maximum blood vessel thickness (20 (pix)), and multiplies the acquired observation distance 5 (mm) by the ratio 1.25. As a result, the observation distance corresponding to the maximum blood vessel thickness MD calculated by the blood vessel index value calculation section 72 is calculated as 5 (mm) x 1.25 = 6.25 (mm) .

Among the blood vessel index values calculated by the blood vessel index value calculation section 72, the density of blood vessels is the same between a case where the observation distance is long and a case where the observation distance is short. Therefore, the density of blood vessels is a distance-invariant blood vessel index value. On the other hand, on the image, the length of the blood vessel in a case where the observation distance is long is smaller than that in a case where the observation distance is short. Therefore, the length of the blood vessel is not a distance-invariant blood vessel index value. On the other hand, on the image, the thickness of the blood vessel in a case where the observation distance is long is smaller than that in a case where the observation distance is short. Therefore, the maximum blood vessel thickness is not a distance-invariant blood vessel index value. In addition, the number of blood vessels is not a distance-invariant blood vessel index value since the number of blood vessels appearing on the image increases as the observation distance increases.

The image generation section 76 generates a special image signal based on the image signal, the blood vessel index value, and the observation distance. An image using a special image signal is a special image. For example, the image generation section 76 generates a base image signal by performing color conversion processing, color enhancement processing, and structure enhancement processing on the image signal. Then, the image generation section 76 generates a special image signal by overlapping information based on the blood vessel index value with respect to the base image signal. The overlapping processing is for displaying information indicating the blood vessel index value and the observation distance for the base image signal. For example, as shown in Fig. 8, the information indicating the blood vessel index value in the ROI 82 and the observation distance are displayed in an upper right region 100 or the like on the monitor 18. The image generation section 76 may display a region, in which the blood vessel index value is equal to or greater than a predetermined value, in a pseudo color by performing coloring processing on the base image signal in accordance with the blood vessel index value. For example, in the ROI 82, a region 101 where the blood vessel index value is equal to or greater than a predetermined value may be displayed in a pseudo color. The image generation section 76 may display the pseudo color region 101 in a different color according to the blood vessel index value.

The video signal generation unit 66 converts the image signal received from the normal image processing unit 62 or the measurement image processing unit 64 into a video signal for displaying as an image that can be displayed on the monitor 18, and outputs the video signal to the monitor 18 in a sequential manner. Accordingly, on the monitor 18, a normal image is displayed in a case where the normal image signal is input, and a special image is displayed in a case where the special image signal is input.

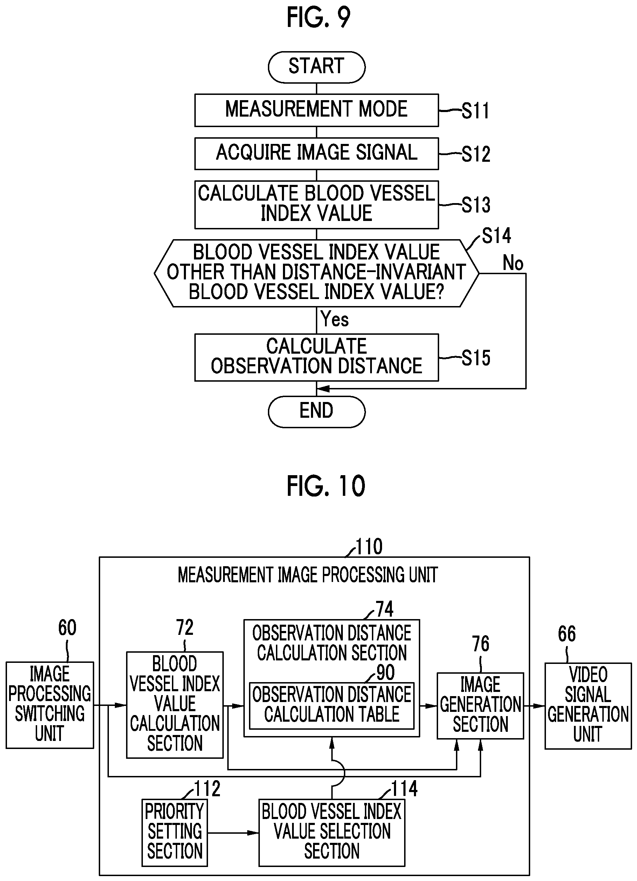

Next, the operation of the present invention will be described with reference to the flowchart shown in Fig. 9. First, in the event that the measurement mode is set (S11), the measurement image processing unit 64 acquires an image signal that is output by imaging the observation target by the imaging sensor 46 (S12). The blood vessel index value calculation section 72 calculates a blood vessel index value from the image signal (S13) . Specifically, the blood vessel index value calculation section 72 calculates the density of blood vessels, the average blood vessel length, and the maximum blood vessel thickness. The observation distance calculation section 74 determines whether or not the density of blood vessels, the average blood vessel length, and the maximum blood vessel thickness are distance-invariant blood vessel index values (S14). The observation distance calculation section 74 determines the average blood vessel length and the maximum blood vessel thickness to be blood vessel index values other than the distance-invariant blood vessel index value (Yes in S14), and calculates the observation distance from the average blood vessel length and the maximum blood vessel thickness (S15) . The observation distance calculation section 74 determines that the density of blood vessels is a distance-invariant blood vessel index value (No in S14), and does not calculate the observation distance for the density of blood vessels.

As described above, in the present invention, a blood vessel index value is calculated from the image signal obtained by imaging the observation target, and the observation distance is calculated based on the blood vessel index value. Therefore, it is possible to acquire the observation distance accurately.

In the embodiment described above, the observation distance calculation section 74 calculates the observation distance from the blood vessel index values other than the distance-invariant blood vessel index value. However, the observation distance may also be calculated from blood vessel index values that are blood vessel index values other than the distance-invariant blood vessel index value and satisfy specific conditions.

For example, as shown in Fig. 10, a measurement image processing unit 110 further includes a priority setting section 112 and a blood vessel index value selection section 114 in addition to the respective components of the measurement image processing unit 64. The priority setting section 112 sets a priority for each blood vessel index value. For example, the priority is set for each blood vessel index value according to the type of a lesion set in advance. In addition, a user, such as a doctor, may set the priority for each blood vessel index value by input using a user interface, such as the console 19.

The blood vessel index value selection section 114 selects a blood vessel index value, for which the priority set by the priority setting section 112 satisfies specific conditions, among the number of blood vessels, the thickness of a blood vessel, the length of a blood vessel, a difference in blood vessel height, the inclination of a blood vessel, the area of a blood vessel, the density of blood vessels, the depth of a blood vessel, and a gap between blood vessels. The observation distance calculation section 74 calculates the observation distance from the blood vessel index value selected by the blood vessel index value selection section 114.

In the embodiment described above, the observation distance calculation section 74 acquires the observation distance based on the blood vessel index value received from the blood vessel index value calculation section 72 and the observation distance and the blood vessel index value associated with the observation distance calculation table 90. However, the method of acquiring the observation distance is not limited thereto. For example, the observation distance calculation section 74 may calculate the observation distance from a predetermined reference observation distance and a reference blood vessel index value at the reference observation distance. Here, in the field of endoscopic observation, diagnostic criteria according to the blood vessel index value at a specific observation distance are determined based on a large number of knowledge obtained at the clinical field so far. The diagnostic criteria are determined according to each state so that a doctor can determine whether a part under observation is in a normal or abnormal state (lesion) . In the present embodiment, a specific observation distance at which diagnostic criterion indicating that an observed part is in a normal state are determined is used as the reference observation distance. In addition, a blood vessel index value calculated from the image signal obtained by imaging the observation target at the reference observation distance is used as the reference blood vessel index value. A specific observation distance at which diagnostic criterion indicating that an observed part is a lesion are determined may be used as the reference observation distance.

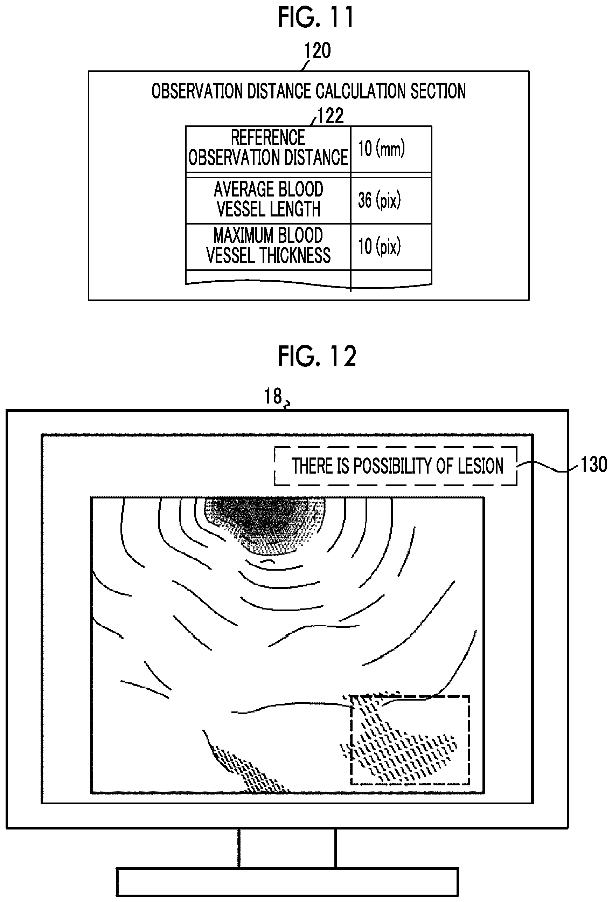

In Fig. 11, a reference storage section 122 is provided in the observation distance calculation section 120. The reference storage section 122 stores a reference observation distance and a reference blood vessel index value at the reference observation distance. In the reference storage section 122, a reference observation distance is stored as DS (mm) and ALs (pix) is stored as an average blood vessel length at the reference observation distance DS (mm). In the reference storage section 122, a maximum blood vessel thickness at the reference observation distance DS (mm) is stored as MDs (pix). For example, the reference observation distance DS is set to 10 (mm), the average blood vessel length ALs at the reference observation distance DS = 10 (mm) is set to 36 (pix), and the maximum blood vessel thickness MDs at the reference observation distance DS = 10 (mm) is set to 10 (pix).

The observation distance calculation section 120 calculates the observation distance by dividing the reference blood vessel index value by the average blood vessel length received from the blood vessel index value calculation section 72 and multiplying a value obtained as a result of the division by the reference observation distance. For example, in a case where the average blood vessel length AL received from the blood vessel index value calculation section 72 is 72 (pix), the reference observation distance DS is 10 (mm) and the average blood vessel length ALs is 36 (pix). Accordingly, the observation distance is calculated as ALs/AL x DS = 36 (pix)/72 (pix) x 10 (mm) = 5 (mm) by the observation distance calculation section 120. In a case where the received maximum blood vessel thickness MD is 20 (pix), the maximum blood vessel thickness MDs is 10 (pix). Accordingly, the observation distance is calculated as MDs/MD x DS = 10 (pix) /20 (pix) x 10 (mm) = 5 (mm) by the observation distance calculation section 120.

In the embodiment described above, for example, in the case of calculating the average blood vessel length, the blood vessel index value calculation section 72 calculates the length of the blood vessel for all pixels in the ROI. Accordingly, a histogram showing the frequency of the length of the blood vessel is obtained. In the histogram showing the frequency of the length of the blood vessel, the horizontal axis is the length of the blood vessel, and the vertical axis is the frequency. Based on the histogram, the observation distance calculation section 74 may determine whether the observation target is in a normal state or is a lesion.

Specifically, a normal histogram showing the frequency of the blood vessel index value in the event that the observation target is in a normal state and an abnormal histogram showing the frequency of the blood vessel index value in the event that the observation target is in an abnormal state, such as a lesion, are stored in advance in the observation distance calculation section 74. Then, the observation distance calculation section 74 determines which of the normal histogram and the abnormal histogram the histogram received from the blood vessel index value calculation section 72 is similar to. As a result of the determination, in a case where the histogram received from the blood vessel index value calculation section 72 is similar to the normal histogram, the observation distance calculation section 74 determines that the observation target is in a normal state. In a case where the histogram received from the blood vessel index value calculation section 72 is similar to the abnormal histogram, the observation distance calculation section 74 determines that the observation target is a potential lesion part, which may be a lesion.

The observation distance calculation section 74 calculates the observation distance in a case where the observation target is in a normal state, and does not calculate the observation distance in a case where the observation target is a potential lesion part. The observation distance calculation section 74 may transmit a determination result to the image generation section 76. In a case where the result of determination that the observation target is a lesion is received, the image generation section 76 generates a special image signal by making information 130 indicating that there is a possibility of a lesion overlap the base image signal. For example, in Fig. 12, as the information 130 indicating that there is a possibility of a lesion, "there is a possibility of a lesion" is displayed in an upper right region or the like on the monitor 18.

### [Second embodiment]

In a second embodiment, the observation target is illuminated using a laser light source and a phosphor instead of the LEDs 20a to 20d of four colors shown in the first embodiment. Since others are the same as in the first embodiment, the explanation thereof will be omitted.

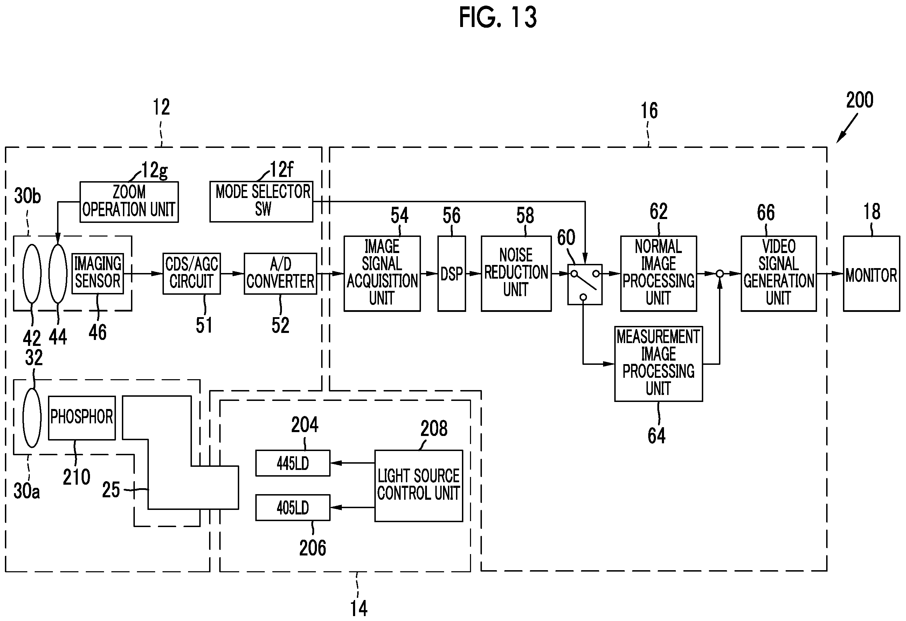

As shown in Fig. 13, in an endoscope system 200 of the second embodiment, instead of the LEDs 20a to 20d of the first embodiment, a blue laser light source (denoted as "445LD" in Fig. 13) 204 and a blue-violet laser light source (denoted as "405LD" in Fig. 13) 206 are provided in the light source device 14. The blue laser light source 204 emits blue laser light having a center wavelength of 445 + 10 nm. The blue-violet laser light source 206 emits blue-violet laser light having a center wavelength of 405 + 10 nm. Emission of light from the blue laser light source 204 and the blue-violet laser light source 206 is individually controlled by a light source control unit 208, so that the light amount ratio between light emitted from the blue laser light source 204 and light emitted from the blue-violet laser light source 206 can be freely changed.

The light source control unit 208 drives the blue laser light source 204 in the case of a normal mode. On the other hand, in the case of a measurement mode, both the blue laser light source 204 and the blue-violet laser light source 206 are driven, and the amount of blue laser light is set to be larger than the amount of blue-violet laser light. The laser light emitted from the blue laser light source 204 and the blue-violet laser light source 206 is incident on the light guide 25 through optical members such as a condensing lens, an optical fiber, and a multiplexer (none is shown).

It is preferable that the half-width of blue laser light or blue-violet laser light is set to about +10 nm. As the blue laser light source 204 and the blue-violet laser light source 206, a broad area type InGaN-based laser diode can be used, and an InGaNAs-based laser diode or a GaNAs-based laser diode can be used. As the light sources, a structure using a light emitter, such as a light emitting diode, may be used.

In addition to the illumination lens 32, a phosphor 210 on which blue laser light or blue-violet laser light from the light guide 25 is incident is provided in the illumination optical system 30a. The phosphor 210 is excited by the blue laser light and emits fluorescence. A part of the blue laser light is transmitted without exciting the phosphor 210. The blue-violet laser light is transmitted without exciting the phosphor 210. The light emitted from the phosphor 210 illuminates the inside of the body of the observation target through the illumination lens 32.

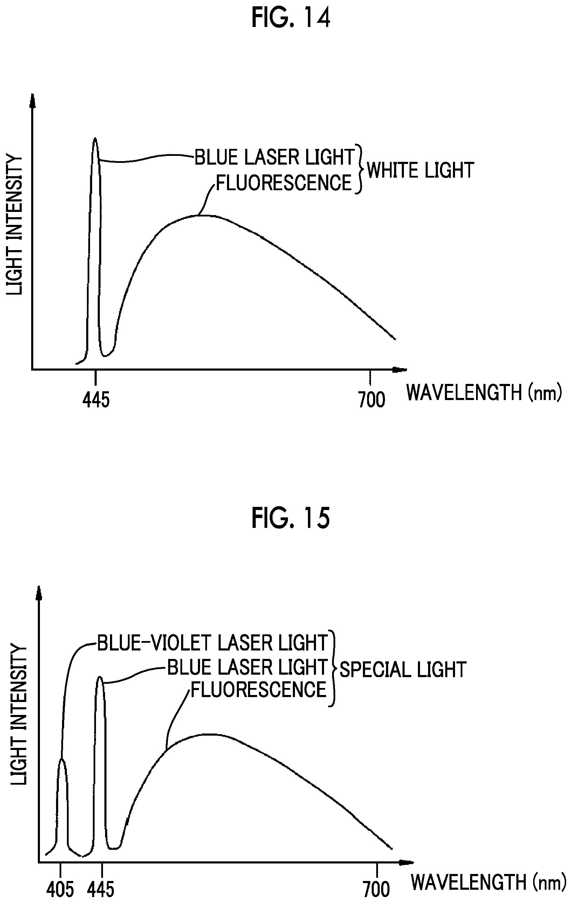

In the normal mode, the blue laser light is mainly incident on the phosphor 210. Accordingly, the observation target is illuminated with white light obtained by combining the blue laser light and the fluorescence as shown in Fig. 14. On the other hand, in the measurement mode, both the blue-violet laser light and the blue laser light are incident on the phosphor 210. Accordingly, the observation target is illuminated with special light obtained by combining the blue-violet laser light, the blue laser light, and the fluorescence as shown in Fig. 15.

As the phosphor 210, it is preferable to use a phosphor configured to include a plurality of kinds of phosphors (for example, a YAG-based phosphor or a phosphor, such as BAM (BaMgAl₁₀O₁₇)) that absorb a part of blue laser light and are excited to emit green to yellow light beams. Assuming that the semiconductor light emitting element is used as the excitation light source of the phosphor 210 as in this configuration example, high-intensity white light can be obtained with high luminous efficiency. Therefore, it is possible to easily adjust the intensity of white light and to suppress the change in color temperature and chromaticity of white light.

### [Third embodiment]

In a third embodiment, the observation target is illuminated using a broadband light source, such as a xenon lamp, and a rotary filter instead of the LEDs 20a to 20d shown in the first embodiment. In addition, the observation target is imaged using a monochrome imaging sensor instead of the color imaging sensor 46. Others are the same as in the first embodiment described above.

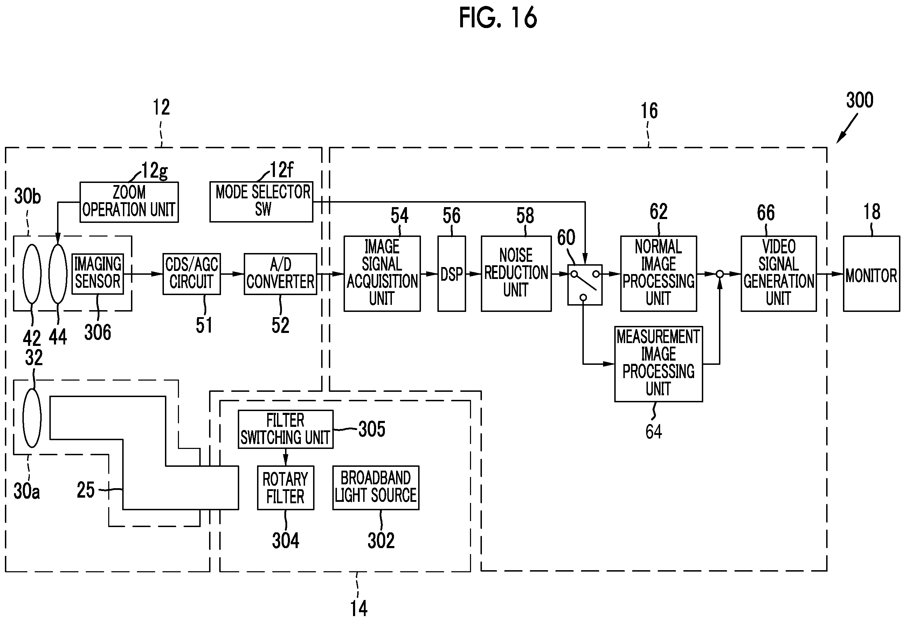

As shown in Fig. 16, in an endoscope system 300 of the third embodiment, instead of the LEDs 20a to 20d, a broadband light source 302, a rotary filter 304, and a filter switching unit 305 are provided in the light source device 14. In addition, instead of the color imaging sensor 46, a monochrome imaging sensor 306 in which no color filter is provided is provided in the imaging optical system 30b.

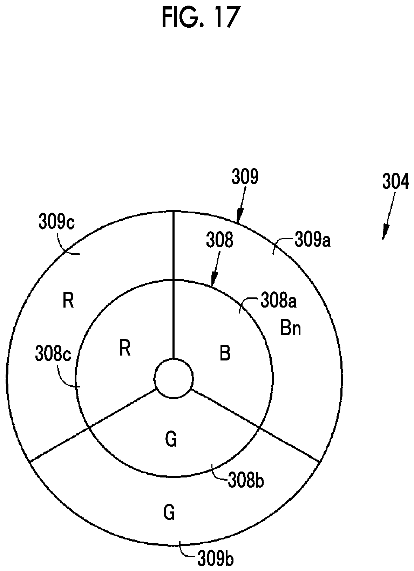

The broadband light source 302 is a xenon lamp, a white LED, or the like, and emits white light having a wavelength range from blue to red. The rotary filter 304 includes a normal mode filter 308 provided on the inner side and a measurement mode filter 309 provided on the outer side (refer to Fig. 17). The filter switching unit 305 moves the rotary filter 304 in the radial direction. The filter switching unit 305 inserts the normal mode filter 308 of the rotary filter 304 in the optical path of white light in a case where the normal mode is set by the mode selector SW 12f, and inserts the measurement mode filter 309 of the rotary filter 304 in the optical path of white light in a case where the measurement mode is set.

As shown in Fig. 17, a B filter 308a, a G filter 308b, and an R filter 308c are provided along the circumferential direction in the normal mode filter 308. The B filter 308a transmits blue light of the white light. The G filter 308b transmits green light of the white light. The R filter 308c transmits red light of the white light. Therefore, at the time of normal mode, the rotary filter 304 rotates to alternately emit the blue light, the green light, and the red light to the observation target.

A Bn filter 309a, a G filter 309b, and an R filter 309c are provided along the circumferential direction in the measurement mode filter 309. The Bn filter 309a transmits blue narrowband light having a specific wavelength of the white light. The G filter 309b transmits green light of the white light. The R filter 309c transmits red light of the white light. Therefore, at the time of measurement mode, the rotary filter 304 rotates to alternately emit the blue narrowband light, the green light, and the red light to the observation target.

In the endoscope system 300, at the time of normal mode, the monochrome imaging sensor 306 images the observation target every time the observation target is illuminated with the blue light, the green light, and the red light. Accordingly, the imaging sensor 306 outputs image signals of RGB. Then, based on the image signals of RGB colors, a normal image is generated using the same method as in the first embodiment described above.

On the other hand, at the time of measurement mode, the monochrome imaging sensor 306 images the observation target every time the observation target is illuminated with the blue narrowband light, the green light, and the red light. Accordingly, the imaging sensor 306 outputs a Bn image signal, a G image signal, and an R image signal. Based on the Bn image signal, the G image signal, and the R image signal, a special image is generated. Thus, in the third embodiment, in order to generate a special image, a Bn image signal is used instead of the B image signal. Other than that, the special image is generated using the same method as in the first embodiment described above.

## Claims

1. A processor device for an endoscope (12), comprising:
an image signal acquisition unit (54) that acquires an image signal obtained by imaging an observation target with the endoscope (12);
a blood vessel index value calculation unit (72) that calculates at least one or more blood vessel index values, among the number of blood vessels, a length of a blood vessel, a difference in blood vessel height, an inclination of a blood vessel, an area of a blood vessel, a density of blood vessels, a depth of a blood vessel, and a gap between blood vessels that are blood vessel index values of blood vessels contained in the observation target, from the image signal; and **characterised in that** the device comprises:
an observation distance calculation unit (74) that calculates an observation distance between a distal end (12d) of the endoscope (12) and a surface of the observation target from at least one or more blood vessel index values of the calculated blood vessel index values; and **in that**:
the blood vessel index values include a distance-invariant blood vessel index value, and the observation distance calculation unit (74) calculates the observation distance from the blood vessel index value other than the distance-invariant blood vessel index value.

2. The processor device for an endoscope (12) according to claim 1,
wherein the blood vessel index value calculation unit (72) further calculates a thickness of a blood vessel as the blood vessel index value, and
the observation distance calculation unit (74) calculates the observation distance from at least two or more of the blood vessel index values among the blood vessel thickness and blood vessel index values other than the blood vessel thickness.

3. The processor device for an endoscope (12) according to claim 2, further comprising:
a blood vessel index value selection unit (114) that selects the blood vessel index value used in calculation of the observation distance among the blood vessel index values.

4. The processor device for an endoscope (12) according to claim 3, further comprising:
a priority setting unit (112) that sets a priority for each of the blood vessel index values,
wherein the blood vessel index value selection unit (114) selects the blood vessel index value, for which the set priority satisfies specific conditions, as the blood vessel index value used in calculation of the observation distance.

5. The processor device for an endoscope (12) according to any one of claims 1 to 4,
wherein the observation distance calculation unit (74) sets an average value, which is obtained by averaging the observation distance for each of the blood vessel index values, as the observation distance.

6. The processor device for an endoscope (12) according to any one of claims 1 to 4, further comprising:
a reference storage unit (122) that stores a predetermined reference observation distance and a reference blood vessel index value at the reference observation distance,
wherein the observation distance calculation unit (74) calculates the observation distance from the calculated blood vessel index value, the reference blood vessel index value, and the reference observation distance.

7. The processor device for an endoscope (12) according to claim 1,
wherein the distance-invariant blood vessel index value is invariant with respect to the observation distance.

8. The processor device for an endoscope (12) according to claim 7,
wherein the distance-invariant blood vessel index value is the density.

9. An operation method of a processor device for an endoscope (12), comprising:
a step in which an image signal acquisition unit (54) acquires an image signal obtained by imaging an observation target with the endoscope (12);
a step in which a blood vessel index value calculation unit (72) calculates at least one or more blood vessel index values, among the number of blood vessels, a length of a blood vessel, a difference in blood vessel height, an inclination of a blood vessel, an area of a blood vessel, a density of blood vessels, a depth of a blood vessel, and a gap between blood vessels that are blood vessel index values of blood vessels contained in the observation target, from the image signal; and **characterised in that** the method comprises:
a step in which an observation distance calculation unit (74) calculates an observation distance between a distal end (12d) of the endoscope (12) and a surface of the observation target from at least one or more blood vessel index values of the calculated blood vessel index values; and **in that**:
the blood vessel index values include a distance-invariant blood vessel index value, and the observation distance calculation unit (74) calculates the observation distance from the blood vessel index value other than the distance-invariant blood vessel index value.

10. A non-transitory computer readable medium storing a control program for an endoscope (12) that is installed in a processor device for the endoscope (12), the program causing a computer to function as:
an image signal acquisition unit (54) that acquires an image signal obtained by imaging an observation target with the endoscope (12);
a blood vessel index value calculation unit (72) that calculates at least one or more blood vessel index values, among the number of blood vessels, a length of a blood vessel, a difference in blood vessel height, an inclination of a blood vessel, an area of a blood vessel, a density of blood vessels, a depth of a blood vessel, and a gap between blood vessels that are blood vessel index values of blood vessels contained in the observation target, from the image signal; and **characterised in that** the program causes the computer to function as:
an observation distance calculation unit (74) that calculates an observation distance between a distal end (12d) of the endoscope (12) and a surface of the observation target from at least one or more blood vessel index values of the calculated blood vessel index values; and **in that**:
the blood vessel index values include a distance-invariant blood vessel index value, and the observation distance calculation unit (74) calculates the observation distance from the blood vessel index value other than the distance-invariant blood vessel index value.

## Patentansprüche

1. Prozessorvorrichtung für ein Endoskop (12), umfassend:
eine Bildsignalerfassungseinheit (54), die ein Bildsignal erfasst, das durch Abbilden eines Beobachtungsziels mit dem Endoskop (12) erhalten wird;
eine Blutgefäßindexwert-Berechnungseinheit (72), die mindestens einen oder mehrere Blutgefäßindexwerte aus der Anzahl von Blutgefäßen, eine Länge eines Blutgefäßes, eine Differenz in der Blutgefäßhöhe, eine Neigung eines Blutgefäßes, eine Fläche eines Blutgefäßes, eine Dichte von Blutgefäßen, eine Tiefe eines Blutgefäßes und eine Lücke zwischen Blutgefäßen, die Blutgefäßindexwerte von Blutgefäßen sind, die in dem Beobachtungsziel enthalten sind, aus dem Bildsignal berechnet; und **dadurch gekennzeichnet, dass** die Vorrichtung umfasst:
eine Beobachtungsabstands-Berechnungseinheit (74), die einen Beobachtungsabstand zwischen einem distalen Ende (12d) des Endoskops (12) und einer Oberfläche des Beobachtungsziels aus mindestens einem oder mehreren Blutgefäßindexwerten der berechneten Blutgefäßindexwerte berechnet; und dadurch, dass:
die Blutgefäßindexwerte einen abstandsinvarianten Blutgefäßindexwert einschließen und die Beobachtungsabstands-Berechnungseinheit (74) den Beobachtungsabstand von dem anderen Blutgefäßindexwert als dem abstandsinvarianten Blutgefäßindexwert berechnet.

2. Prozessorvorrichtung für ein Endoskop (12) nach Anspruch 1,
wobei die Blutgefäßindexwert-Berechnungseinheit (72) weiterhin eine Dicke eines Blutgefäßes als den Blutgefäßindexwert berechnet, und
die Beobachtungsabstands-Berechnungseinheit (74) den Beobachtungsabstand aus mindestens zwei oder mehreren der Blutgefäßindexwerte aus der Blutgefäßdicke und anderen Blutgefäßindexwerten als der Blutgefäßdicke berechnet.

3. Prozessorvorrichtung für ein Endoskop (12) nach Anspruch 2, weiterhin umfassend:
eine Blutgefäßindexwert-Auswahleinheit (114), die den Blutgefäßindexwert auswählt, der bei der Berechnung des Beobachtungsabstands unter den Blutgefäßindexwerten verwendet wird.

4. Prozessorvorrichtung für ein Endoskop (12) nach Anspruch 3, weiterhin umfassend:
eine Prioritätseinstelleinheit (112), die eine Priorität für jeden der Blutgefäßindexwerte einstellt,
wobei die Blutgefäßindexwert-Auswahleinheit (114) den Blutgefäßindexwert, für den die eingestellte Priorität spezifische Bedingungen erfüllt, als den Blutgefäßindexwert auswählt, der bei der Berechnung des Beobachtungsabstands verwendet wird.

5. Prozessorvorrichtung für ein Endoskop (12) nach einem der Ansprüche 1 bis 4,
wobei die Beobachtungsabstands-Berechnungseinheit (74) einen Mittelwert, der durch Mitteln des Beobachtungsabstands für jeden der Blutgefäßindexwerte erhalten wird, als Beobachtungsabstand festlegt.

6. Prozessorvorrichtung für ein Endoskop (12) nach einem der Ansprüche 1 bis 4, weiterhin umfassend:
eine Referenzspeichereinheit (122), die einen vorbestimmten Referenzbeobachtungsabstand und einen Referenzblutgefäßindexwert in dem Referenzbeobachtungsabstand speichert,
wobei die Beobachtungsabstands-Berechnungseinheit (74) den Beobachtungsabstand aus dem berechneten Blutgefäßindexwert, dem Referenzblutgefäßindexwert und dem Referenzbeobachtungsabstand berechnet.

7. Prozessorvorrichtung für ein Endoskop (12) nach Anspruch 1,
wobei der abstandsinvariante Blutgefäßindexwert in Bezug auf den Beobachtungsabstand invariant ist.

8. Prozessorvorrichtung für ein Endoskop (12) nach Anspruch 7,
wobei der abstandsinvariante Blutgefäßindexwert die Dichte ist.

9. Betriebsverfahren einer Prozessorvorrichtung für ein Endoskop (12), umfassend:
einen Schritt, bei dem eine Bildsignalerfassungseinheit (54) ein Bildsignal erfasst, das durch Abbilden eines Beobachtungsziels mit dem Endoskop (12) erhalten wird;
einen Schritt, bei dem eine Blutgefäßindexwert-Berechnungseinheit (72) mindestens einen oder mehrere Blutgefäßindexwerte aus der Anzahl von Blutgefäßen, eine Länge eines Blutgefäßes, eine Differenz in der Blutgefäßhöhe, eine Neigung eines Blutgefäßes, eine Fläche eines Blutgefäßes, eine Dichte von Blutgefäßen, eine Tiefe eines Blutgefäßes und eine Lücke zwischen Blutgefäßen, die Blutgefäßindexwerte von Blutgefäßen sind, die in dem Beobachtungsziel enthalten sind, aus dem Bildsignal berechnet; und **dadurch gekennzeichnet, dass** das Verfahren umfasst:
einen Schritt, bei dem eine Beobachtungsabstands-Berechnungseinheit (74) einen Beobachtungsabstand zwischen einem distalen Ende (12d) des Endoskops (12) und einer Oberfläche des Beobachtungsziels aus mindestens einem oder mehreren Blutgefäßindexwerten der berechneten Blutgefäßindexwerte berechnet; und dadurch, dass:
die Blutgefäßindexwerte einen abstandsinvarianten Blutgefäßindexwert einschließen und die Beobachtungsabstands-Berechnungseinheit (74) den Beobachtungsabstand von dem anderen Blutgefäßindexwert als dem abstandsinvarianten Blutgefäßindexwert berechnet.

10. Nichtflüchtiges computerlesbares Medium, das ein Steuerprogramm für ein Endoskop (12) speichert, das in einer Prozessorvorrichtung für das Endoskop (12) installiert ist, wobei das Programm bewirkt, dass ein Computer fungiert als:
eine Bildsignalerfassungseinheit (54), die ein Bildsignal erfasst, das durch Abbilden eines Beobachtungsziels mit dem Endoskop (12) erhalten wird;
eine Blutgefäßindexwert-Berechnungseinheit (72), die mindestens einen oder mehrere Blutgefäßindexwerte aus der Anzahl von Blutgefäßen, eine Länge eines Blutgefäßes, eine Differenz in der Blutgefäßhöhe, eine Neigung eines Blutgefäßes, eine Fläche eines Blutgefäßes, eine Dichte von Blutgefäßen, eine Tiefe eines Blutgefäßes und eine Lücke zwischen Blutgefäßen, die Blutgefäßindexwerte von Blutgefäßen sind, die in dem Beobachtungsziel enthalten sind, aus dem Bildsignal berechnet; und **dadurch gekennzeichnet, dass** das Programm bewirkt, dass der Computer fungiert als:
eine Beobachtungsabstands-Berechnungseinheit (74), die einen Beobachtungsabstand zwischen einem distalen Ende (12d) des Endoskops (12) und einer Oberfläche des Beobachtungsziels aus mindestens einem oder mehreren Blutgefäßindexwerten der berechneten Blutgefäßindexwerte berechnet; und dadurch, dass:
die Blutgefäßindexwerte einen abstandsinvarianten Blutgefäßindexwert einschließen und die Beobachtungsabstands-Berechnungseinheit (74) den Beobachtungsabstand von dem anderen Blutgefäßindexwert als dem abstandsinvarianten Blutgefäßindexwert berechnet.

## Revendications

1. Dispositif de processeur pour un endoscope (12), comprenant :
une unité d'acquisition de signal d'image (54) qui acquiert un signal d'image obtenu par imagerie d'une cible d'observation avec l'endoscope (12) ;
une unité de calcul de valeur d'indice de vaisseau sanguin (72) qui calcule au moins une ou plusieurs valeurs d'indice de vaisseau sanguin, parmi le nombre de vaisseaux sanguins, une longueur d'un vaisseau sanguin, une différence de hauteur de vaisseau sanguin, une inclinaison d'un vaisseau sanguin, une superficie d'un vaisseau sanguin, une densité de vaisseaux sanguins, une profondeur d'un vaisseau sanguin, et un écart entre des vaisseaux sanguins qui sont des valeurs d'indice de vaisseau sanguin de vaisseaux sanguins contenus dans la cible d'observation, à partir du signal d'image ; et **caractérisé en ce que** le dispositif comprend :
une unité de calcul de distance d'observation (74) qui calcule une distance d'observation entre une extrémité distale (12d) de l'endoscope (12) et une surface de la cible d'observation à partir d'au moins une ou plusieurs valeurs d'indice de vaisseau sanguin des valeurs d'indice de vaisseau sanguin calculées ; et **en ce que** :
les valeurs d'indice de vaisseau sanguin incluent une valeur d'indice de vaisseau sanguin invariante en distance, et l'unité de calcul de distance d'observation (74) calcule la distance d'observation à partir de la valeur d'indice de vaisseau sanguin autre que la valeur d'indice de vaisseau sanguin invariante en distance.

2. Dispositif de processeur pour un endoscope (12) selon la revendication 1,
dans lequel l'unité de calcul de valeur d'indice de vaisseau sanguin (72) calcule en outre une épaisseur d'un vaisseau sanguin comme valeur d'indice de vaisseau sanguin, et
l'unité de calcul de distance d'observation (74) calcule la distance d'observation à partir d'au moins deux ou plusieurs des valeurs d'indice de vaisseau sanguin parmi l'épaisseur de vaisseau sanguin et des valeurs d'indice de vaisseau sanguin autres que l'épaisseur de vaisseau sanguin.

3. Dispositif de processeur pour un endoscope (12) selon la revendication 2, comprenant en outre :
une unité de sélection de valeur d'indice de vaisseau sanguin (114) qui sélectionne la valeur d'indice de vaisseau sanguin utilisée dans le calcul de la distance d'observation parmi les valeurs d'indice de vaisseau sanguin.

4. Dispositif de processeur pour un endoscope (12) selon la revendication 3, comprenant en outre :
une unité de définition de priorité (112) qui définit une priorité pour chacune des valeurs d'indice de vaisseau sanguin,
dans lequel l'unité de sélection de valeur d'indice de vaisseau sanguin (114) sélectionne la valeur d'indice de vaisseau sanguin, pour laquelle la priorité définie satisfait à des conditions spécifiques, en tant que valeur d'indice de vaisseau sanguin utilisée dans le calcul de la distance d'observation.

5. Dispositif de processeur pour un endoscope (12) selon l'une quelconque des revendications 1 à 4,
dans lequel l'unité de calcul de distance d'observation (74) définit une valeur moyenne, qui est obtenue par établissement de la moyenne de la distance d'observation pour chacune des valeurs d'indice de vaisseau sanguin, en tant que distance d'observation.

6. Dispositif de processeur pour un endoscope (12) selon l'une quelconque des revendications 1 à 4, comprenant en outre :
une unité de stockage de référence (122) qui stocke une distance d'observation de référence prédéterminée et une valeur d'indice de vaisseau sanguin de référence à la distance d'observation de référence,
dans lequel l'unité de calcul de distance d'observation (74) calcule la distance d'observation à partir de la valeur d'indice de vaisseau sanguin calculée, de la valeur d'indice de vaisseau sanguin de référence, et de la distance d'observation de référence.

7. Dispositif de processeur pour un endoscope (12) selon la revendication 1,
dans lequel la valeur d'indice de vaisseau sanguin invariante en distance est invariante par rapport à la distance d'observation.

8. Dispositif de processeur pour un endoscope (12) selon la revendication 7,
dans lequel la valeur d'indice de vaisseau sanguin invariante en distance est la densité.

9. Procédé de fonctionnement d'un dispositif de processeur pour un endoscope (12), comprenant :
une étape dans laquelle une unité d'acquisition de signal d'image (54) acquiert un signal d'image obtenu par imagerie d'une cible d'observation avec l'endoscope (12) ;
une étape dans laquelle une unité de calcul de valeur d'indice de vaisseau sanguin (72) calcule au moins une ou plusieurs valeurs d'indice de vaisseau sanguin, parmi le nombre de vaisseaux sanguins, une longueur d'un vaisseau sanguin, une différence de hauteur de vaisseau sanguin, une inclinaison d'un vaisseau sanguin, une superficie d'un vaisseau sanguin, une densité de vaisseaux sanguins, une profondeur d'un vaisseau sanguin, et un écart entre des vaisseaux sanguins qui sont des valeurs d'indice de vaisseau sanguin de vaisseaux sanguins contenus dans la cible d'observation, à partir du signal d'image ; et **caractérisé en ce que** le procédé comprend :
une étape dans laquelle une unité de calcul de distance d'observation (74) calcule une distance d'observation entre une extrémité distale (12d) de l'endoscope (12) et une surface de la cible d'observation à partir d'au moins une ou plusieurs valeurs d'indice de vaisseau sanguin des valeurs d'indice de vaisseau sanguin calculées ; et **en ce que** :
les valeurs d'indice de vaisseau sanguin incluent une valeur d'indice de vaisseau sanguin invariante en distance, et l'unité de calcul de distance d'observation (74) calcule la distance d'observation à partir de la valeur d'indice de vaisseau sanguin autre que la valeur d'indice de vaisseau sanguin invariante en distance.

10. Support lisible par ordinateur non transitoire stockant un programme de commande pour un endoscope (12) qui est installé dans un dispositif de processeur pour l'endoscope (12), le programme amenant un ordinateur à fonctionner comme :
une unité d'acquisition de signal d'image (54) qui acquiert un signal d'image obtenu par imagerie d'une cible d'observation avec l'endoscope (12) ;
une unité de calcul de valeur d'indice de vaisseau sanguin (72) qui calcule au moins une ou plusieurs valeurs d'indice de vaisseau sanguin, parmi le nombre de vaisseaux sanguins, une longueur d'un vaisseau sanguin, une différence de hauteur de vaisseau sanguin, une inclinaison d'un vaisseau sanguin, une superficie d'un vaisseau sanguin, une densité de vaisseaux sanguins, une profondeur d'un vaisseau sanguin, et un écart entre des vaisseaux sanguins qui sont des valeurs d'indice de vaisseau sanguin de vaisseaux sanguins contenus dans la cible d'observation, à partir du signal d'image ; et **caractérisé en ce que** le programme amène l'ordinateur à fonctionner comme :
une unité de calcul de distance d'observation (74) qui calcule une distance d'observation entre une extrémité distale (12d) de l'endoscope (12) et une surface de la cible d'observation à partir d'au moins une ou plusieurs valeurs d'indice de vaisseau sanguin des valeurs d'indice de vaisseau sanguin calculées ; et **en ce que** :
les valeurs d'indice de vaisseau sanguin incluent une valeur d'indice de vaisseau sanguin invariante en distance, et l'unité de calcul de distance d'observation (74) calcule la distance d'observation à partir de la valeur d'indice de vaisseau sanguin autre que la valeur d'indice de vaisseau sanguin invariante en distance.
